# EUROPÄISCHE PATENTSCHRIFT

(11) **EP 4 021 544 B1**
(45) Veröffentlichungstag und Bekanntmachung des Hinweises auf die Patenterteilung: **05.11.2025**
(21) Anmeldenummer: 20767482.1
(22) Anmeldetag: 25.08.2020
(51) Int. Cl.: A61M 16/06, A62B 18/08, A41D 13/11, A62B 18/02, A62B 9/02

(54) **SAUERSTOFFMASKE ZUM ZUFÜHREN VON SAUERSTOFF AN EINE PERSON**
OXYGEN MASK FOR SUPPLYING OXYGEN TO A PERSON
MASQUE À OXYGÈNE DESTINÉ À L'ALIMENTATION EN OXYGÈNE D'UNE PERSONNE

(30) Priorität: 30.08.2019 DE 102019123331
(43) Veröffentlichungstag der Anmeldung: 06.07.2022
(73) Patentinhaber: Airbus Operations GmbH, 21129 Hamburg (DE)
(72) Erfinder: KLIEM, Patricia, 21129 Hamburg (DE)
(74) Vertreter: LKGLOBAL Lorenz & Kopf Patentanwalt Attorney at Law PartG mbB
(86) Internationale Anmeldenummer: PCT/EP2020/073710
(87) Internationale Veröffentlichungsnummer: WO 2021/037830

(56) Entgegenhaltungen:
- WO-A1-2009/038934
- GB-A- 697 762

## Beschreibung

### TECHNISCHES GEBIET

Die Erfindung betrifft eine Sauerstoffmaske zum Zuführen von Sauerstoff an eine Person, ein System zum Bereitstellen von Sauerstoff an Sauerstoffmasken in einem Luftfahrzeug sowie ein Luftfahrzeug mit einer Passagierkabine und mindestens einem derartigen System.

### HINTERGRUND DER ERFINDUNG

Sauerstoffmasken in Verkehrsflugzeugen sind üblicherweise für die Verwendung von erwachsenen Personen ausgelegt. Bei einem System zum Bereitstellen von Sauerstoff mit einem kontinuierlichen Sauerstofffluss wird eine mit Sauerstoff angereicherte Umgebung innerhalb der Sauerstoffmaske auf einer zu der Person gerichteten Innenseite erzeugt. Auch wenn aufgrund von Leckagen die Luft aus der Sauerstoffmaske nach außen treten kann, erhält der Benutzer dennoch eine ausreichende Menge Sauerstoff.

Bei moderneren Sauerstoffsystemen könnte es jedoch erforderlich werden, weitgehend leckagefreie Sauerstoffmasken einzusetzen, so dass eine Person mit einem nach EASA Zulassungsrichtlinien CS25 ausreichenden Sauerstofffluss versorgt werden kann. Dies bedeutet, dass die Sauerstoffmaske dicht an dem Gesicht der betreffenden Person anliegen muss. Insbesondere für Babys, Kleinkinder und Personen mit Bart können somit angepasste Sauerstoffmasken sinnvoll sein.

GB 697 762 A offenbart eine Gesichtsmaske, umfassend einen Beutel aus flexiblem, gasdichtem Material, in dessen offene Seite die Nase und das Kinn einer Person einzuführen sind und Mittel zum Halten des Beutels auf dem Gesicht des Trägers, wobei eine Seite der Öffnung fest unter dem Kinn gehalten wird. Weiterhin ist ein verformbarer, unelastischer Draht oder Stab vorgesehen, der sich entlang der anderen Seite der Öffnung erstreckt und geeignet ist, gebogen zu werden, um diesen Rand des Beutels in Übereinstimmung mit der Kontur des Gesichts des Trägers zu formen. Der Beutel weist einen Einlass zum Verbinden des Beutels mit einer Gasversorgung und Auslassöffnungen in der Wand des Beutels auf.

WO 2009/038934 A1 offenbart eine Filtergesichtsmaske, aufweisend ein Gurtzeug und einen Maskenkörper. Der Maskenkörper umfasst eine Filterstruktur und eine Stützstruktur. Die Stützstruktur umfasst erste und zweite gegenüberliegende Seitenteile, die jeweils ein bewegliches Scharnier aufweisen. Durch die Verwendung von beweglichen Scharnieren kann der Maskenkörper dynamisch auf die Kieferbewegung des Trägers reagieren.

### ZUSAMMENFASSUNG DER ERFINDUNG

Es ist folglich eine Aufgabe der Erfindung, eine Sauerstoffmaske vorzuschlagen, die ohne Modifikationen an unterschiedliche Gesichtsgrößen und einen Bart angepasst werden kann, ohne dass im Bedarfsfall eine zusätzliche Maske beschafft werden muss.

Die Aufgabe wird durch eine Sauerstoffmaske mit den Merkmalen des unabhängigen Anspruchs 1 gelöst. Vorteilhafte Ausführungsformen und Weiterbildungen sind den Unteransprüchen und der nachfolgenden Beschreibung zu entnehmen.

Es wird eine Sauerstoffmaske zum Zuführen von Sauerstoff an eine Person vorgeschlagen, aufweisend einen Halterahmen, der mindestens einen Quersteg aufweist, einen auf dem Halterahmen angeordneten und von dem mindestens einen Quersteg aufspannbaren, im Wesentlichen gasundurchlässigen Stoff, ein mit dem Halterahmen verbundenes Kopfband, ein an dem Stoff angeordnetes und den Stoff durchdringendes Ausatemventil, und eine Sauerstoffzuführungsleitung zum Bereitstellen von Sauerstoff an einer Innenseite der Sauerstoffmaske, wobei der mindestens eine Quersteg in zwei einander gegenüberliegenden seitlichen Haltern angeordnet ist, wobei der mindestens eine Quersteg flexibel ist, und wobei der Abstand der seitlichen Halter zueinander zumindest zwischen einem ersten und einem zweiten Abstand einstellbar ist.

Die erfindungsgemäße Sauerstoffmaske besteht folglich aus zwei wesentlichen Komponenten, die durch ein Ausatemventil und eine Sauerstoffzuführungsleitung ergänzt werden. Ein Kernprinzip liegt in dem Aufspannen eines Stoffs mithilfe eines Halterahmens, der mindestens einen Quersteg aufweist. Ein Quersteg ist eine Komponente, welche in einer Querrichtung der Sauerstoffmaske verläuft. Dies bedeutet, dass sich ein Quersteg von einer Seite der Sauerstoffmaske zu einer gegenüberliegenden Seite erstreckt. Es kann ein Quersteg eingesetzt werden, der sich etwa über einen Nasenbereich erstreckt. Ein weiterer Quersteg könnte sich in einem Kinnbereich befindet. Werden mehrere Querstege eingesetzt, könnten diese einander ähneln oder unterschiedlich ausgebildet sein und dabei unterschiedliche Querschnitte, unterschiedliche Längen oder unterschiedliche Materialien aufweisen. Ein Quersteg könnte etwa einen deutlich geringeren Querschnitt aufweisen und sehr elastisch sein, ähnlich wie ein Gummiband.

Der mindestens eine Quersteg ist dazu ausgebildet, den Stoff aufzuspannen und zu halten. Das Aufspannen bedeutet dabei nicht, dass der Stoff stramm zwischen mehreren Querstegen gespannt ist. Stattdessen könnte der Stoff auch eine gewölbte und dem Gesicht der Person folgende Form besitzen und der mindestens eine Quersteg unterstützt das Beibehalten dieser Form.

Durch die Flexibilität des mindestens einen Querstegs kann sich die Sauerstoffmaske insbesondere an den Nasen- und Mundbereich anschmiegen. Es ist dabei bevorzugt, dass bei der Verwendung mehrerer Querstege alle Querstege der Sauerstoffmaske flexibel sind. Die Sauerstoffmaske kann dann von einem Benutzer aufgesetzt werden und in alle Raumrichtungen eine ausgeprägte Krümmung bzw. Wölbung einnehmen.

Das Kopfband, welches mit dem Halterahmen verbunden ist, erlaubt das Fixieren der Sauerstoffmaske an dem Kopf des Benutzers. Bevorzugt ist das Kopfband an den seitlichen Haltern angeordnet, so dass diese in einer äußeren Position fixiert werden und das Kopfband den Verlauf des mindestens einen Querstegs nicht beeinflusst. Die seitlichen Halter können demnach eine mechanische Schnittstelle zwischen dem Kopfband und dem Stoff ausbilden.

Das Ausatemventil erlaubt das gezielte Abführen verbrauchter Luft durch einen beim Ausatmen entstehenden Überdruck in der Sauerstoffmaske. Es ist insbesondere von einem Ende der Sauerstoffzuführungsleitung beabstandet, so dass kein ungewünschter Sauerstoffverlust eintritt.

Zur Anpassung der Sauerstoffmaske an die Größe des Gesichts des Benutzers können die seitlichen Halter einen variablen Abstand zueinander einnehmen. Dies bedeutet, dass die Breite der Sauerstoffmaske bedarfsweise vergrößert werden kann. Es bietet sich besonders an, die Sauerstoffmaske mit einer kleinstmöglichen Breite zu verstauen, so dass sie vom Benutzer gegriffen werden kann, um beispielsweise durch eine leichte, intuitive Handbewegung die Sauerstoffmaske zu verbreitern. Es ist denkbar, dies durch Auseinanderziehen der seitlichen Halter durchzuführen. Aufgrund des flexiblen mindestens einen Querstegs kann die vom Stoff überspannte Fläche bedarfsweise ebenso vergrößert werden. Hierzu werden bei Verwendung mehrerer Querstege diese bevorzugt in einer Querrichtung voneinander beanstandet.

Durch die erfindungsgemäße Sauerstoffmaske können unterschiedliche Eigenschaften der Benutzer berücksichtigt werden und die Sauerstoffmaske kann sich bündig und abdichtend an das Gesicht des Benutzers legen. Sowohl Babys, als auch Kleinkinder können optimal mit einer Sauerstoffmaske versorgt werden. Auch Personen mit Bart können die Sauerstoffmaske so anlegen, dass der gesamte Bart von dem Stoff überspannt wird und sich der mindestens eine Quersteg bündig an angrenzende Hautpartien anlegen können. Es sind keine improvisierten Lösungen notwendig und praktisch alle denkbaren Benutzer können die erfindungsgemäße Sauerstoffmaske benutzen. Jeder Sitzplatz kann folglich mit dieser Sauerstoffmaske ausgestattet werden und es müssen keine speziellen Sauerstoffmasken separat mitgeführt werden.

In einer vorteilhaften Ausführungsform ist der mindestens eine Quersteg an oder in den seitlichen Haltern verschiebbar gelagert. Die Verschiebbarkeit verläuft dabei insbesondere entlang der Längserstreckung des betreffenden Querstegs. Dem Benutzer ist es folglich leicht möglich, durch Ziehen der seitlichen Halter seitlich nach außen die Breite der Sauerstoffmaske zu vergrößern. Die verschiebbare Lagerung sollte mechanisch möglichst einfach sein, so dass eine zuverlässige Funktion garantiert werden kann und ein möglichst geringes Mehrgewicht aus dieser Verschiebbarkeit resultiert.

Bevorzugt weist mindestens einer der seitlichen Halter für den mindestens einen Quersteg einen geradlinigen Hohlraum auf, der auf den betreffenden Quersteg angepasst ist, so dass der betreffende Quersteg unterschiedlich tief in den Hohlraum einschiebbar ist. Der seitliche Halter kann folglich den Quersteg mechanisch sehr einfach aufnehmen und umfangsseitig umschließen. Durch das Ziehen des seitlichem Halters in Richtung des Endes des Querstegs kann dieser folglich entlang des Hohlraums rutschen und damit seine effektive Länge vergrößern. Es ist besonders bevorzugt, dass der Quersteg und/oder der Hohlraum mit einer einen hohen Reibungskoeffizienten aufweisenden Oberfläche versehen ist und/oder eine so enge Passung gewählt wird, dass ein Verschieben nur durch das Überwinden eines gewissen Widerstands möglich wird und folglich der Quersteg in einer verschobenen Position verbleibt.

Der Hohlraum und der betreffende Quersteg könnten in einer vorteilhaften Ausführungsform eine Begrenzungseinrichtung aufweisen, die dazu ausgebildet ist, ein vollständiges Herausziehen des Querstegs zu verhindern. Insbesondere wenn eine größere Zugkraft zum Herausziehen vorgesehen ist, um die Breite zu vergrößern, sollte verhindert werden, dass der Quersteg unbeabsichtigt vollständig aus dem Hohlraum herausrutscht. Durch die Begrenzungseinrichtung kann eine Art mechanischer Anschlag realisiert werden, der auch eine haptische Rückmeldung darüber gibt, dass die maximale Breite erreicht ist.

Besonders bevorzugt ist die Begrenzungseinrichtung in Form einer endseitigen Aufdickung des betreffenden Querstegs und einer endseitigen Verjüngung des Hohlraums gebildet. Die endseitige Aufdeckung gerät in einer Endlage mit der Verjüngung des Hohlraums in einen Flächenkontakt, so dass ein weiteres Verschieben verhindert wird.

Bevorzugt ist der Stoff zwischen einem ersten Quersteg und einem zweiten Quersteg aufspannbar. Der erste Quersteg könnte etwa für den Nasenbereich vorgesehen sein, der zweite Quersteg für den Kinnbereich der Person. Beide Querstege könnten einen gleichen oder ähnlichen Querschnitt und ein ähnliches oder gleiches Material aufweisen. Es wäre insbesondere auch denkbar, einen des ersten Querstegs und des zweiten Querstegs auch wie eine Art Gummiband oder ein anderes elastisches Element auszubilden. Dieses könnte fest mit den seitlichen Haltern oder einer Stoffkante verbunden sein.

Die Sauerstoffmaske könnte einen dritten Quersteg aufweisen, der zwischen dem ersten Quersteg und dem zweiten Quersteg angeordnet ist. Der dritte Quersteg ist in Querrichtung folglich zwischen dem ersten und dem zweiten Quersteg angeordnet. Er könnte der Sauerstoffmaske eine größere Stabilität verleihen.

Die seitlichen Halter könnten weiterhin aus einem elastischen Material gebildet sein. Das elastische Material verleiht auch den seitlichen Haltern eine gewisse Formbarkeit, um die Sauerstoffmaske ideal an den betreffenden Benutzer anzupassen. Es ist vorstellbar, dass die seitlichen Halter aus demselben Material hergestellt sind, wie die Querstege.

Es ist bevorzugt, dass der mindestens eine Quersteg bereichsweise eine vorgeformte Krümmung aufweist, die zum Anschmiegen an die Nase der Person geformt ist. Die vorgeformte Krümmung kann nicht nur zur verbesserten Anpassung der Form der Sauerstoffmaske genutzt werden, sondern könnte einer Person auch intuitiv anzeigen, wie die Sauerstoffmaske aufzusetzen ist.

Es kann sich weiterhin auch anbieten, dass der mindestens eine Quersteg insgesamt leicht gekrümmt ist, so dass eine vorgesehene, der Person zugewandte und insbesondere konkav geformte Innenseite eingenommen und unmittelbar erkannt wird.

In einer vorteilhaften Ausführungsform weisen die seitlichen Halter mehrere Halteabschnitte zum Aufnehmen jeweils eines Querstegs auf, wobei die Halteabschnitte an einem Gelenk angeordnet sind, das dazu ausgebildet ist, die Halteabschnitte zum Anpassen an die Person variabel zueinander aufgefächert zu werden. Die Halteabschnitte können zum Anpassen an den Benutzer auf gewünschte Weise aufgefächert werden, so dass der Stoff eine mehr oder weniger große Fläche überspannt. Das Gelenk kann sehr einfach ausgeführt sein und sich auf ein Sicherungselement beschränken, das die einzelnen Halteabschnitte an einem gemeinsamen Punkt durchsetzt. Das Sicherungselement könnte beispielsweise ein stabförmiges Element sein, das insbesondere aus demselben Material hergestellt ist, wie die seitlichen Halter.

Es ist bevorzugt, wenn die seitlichen Halter und der mindestens eine Quersteg aus Silikon, einem silikonartigen Material, einem Elastomer oder einem gummiartigen Material ausgebildet sind. Dadurch ist der mindestens eine Quersteg sehr flexibel und elastisch und passt sich unmittelbar beim Aufsetzen auf die betreffende Kopfform des Benutzers an.

Zum Verstellen des Kopfbandes kann es sich anbieten, wenn das Kopfband zwei steife Endabschnitte aufweist, die jeweils in einer verzahnten Vertiefung stecken, wobei das Kopfband und die verzahnten Vertiefungen dazu ausgebildet sind, eine effektive Länge des Kopfbands inkrementell zu ändern. Eine Verzahnung könnte insbesondere mit Rillen an den steifen Endabschnitten interagieren, so dass durch eine Zugkraft auf das Kopfband einzelne Rillen durch die Verzahnung übersprungen werden können und stets eine zumindest leichte Arretierung in mindestens einer Rille vorliegt. Da die Endabschnitte steif sind, ist zusätzlich zu einer Zugbewegung auch das Eindrücken der Endabschnitte in die zugehörigen Vertiefungen denkbar. Die effektive Länge des Kopfbandes könnte so sowohl vergrößert, als auch verkleinert werden.

Die Sauerstoffzuführungsleitung könnte weiterhin durch den Stoff zu der Innenseite der Sauerstoffmaske ragen. Die Öffnung der Sauerstoffzuführungsleitung könnte dabei bündig mit dem Stoff an der Innenseite enden.

Die Erfindung betrifft ferner ein System zum Bereitstellen von Sauerstoff an Sauerstoffmasken in einem Luftfahrzeug. Das System weist eine Sauerstoffquelle und eine oder mehrere Sauerstoffmasken nach einem der vorhergehenden Ansprüche auf, wobei die Sauerstoffzuführungsleitung der Sauerstoffmasken jeweils mit der Sauerstoffquelle verbindbar sind.

Weiterhin betrifft die Erfindung ein Luftfahrzeug, aufweisend eine Passagierkabine und mindestens ein vorangehend dargestelltes System.

### KURZE BESCHREIBUNG DER FIGUREN

Weitere Merkmale, Vorteile und Anwendungsmöglichkeiten der vorliegenden Erfindung ergeben sich aus der nachfolgenden Beschreibung der Ausführungsbeispiele und den Figuren. Dabei bilden alle beschriebenen und/oder bildlich dargestellten Merkmale für sich und in beliebiger Kombination den Gegenstand der Erfindung auch unabhängig von ihrer Zusammensetzung in den einzelnen Ansprüchen oder deren Rückbezügen. In den Figuren stehen weiterhin gleiche Bezugszeichen für gleiche oder ähnliche Objekte.
Fig. 1 zeigt eine Sauerstoffmaske in einer Vorderansicht.
Fig. 2 zeigt verschiedene Zustände der Querstege in drei aufeinanderfolgenden schematischen Darstellungen.
Fig. 3 zeigt einen schematischen Detailschnitt in seitlichen Haltern und einem Quersteg.
Fig. 4 zeigt eine schematische Darstellung von gelenkig gehaltenen Querstegen.
Fig. 5 zeigt in zwei Ansichten das Kopfband in zwei unterschiedlichen Positionen.
Fig. 6 zeigt einen schematischen Detailschnitt der Anbindung des Kopfbandes an den seitlichen Haltern.
Fig. 7 zeigt ein Flugzeug, in dem Sauerstoffmasken vorgesehen sind.

### DETAILLIERTE DARSTELLUNG EXEMPLARISCHER AUSFÜHRUNGSFORMEN

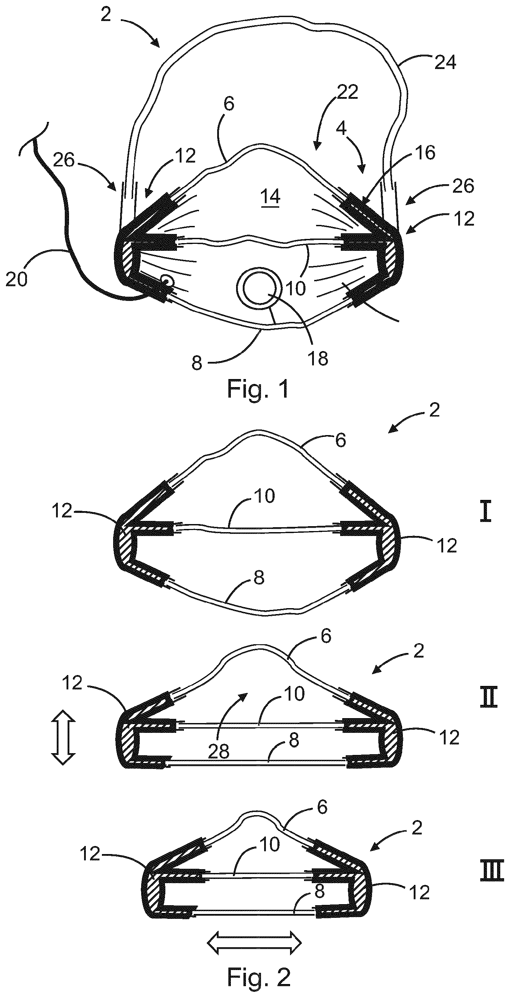

Fig. 1 zeigt eine Sauerstoffmaske 2 zum Zuführen von Sauerstoff an eine Person. Die Sauerstoffmaske 2 kann insbesondere in einer Kabine eines Verkehrsmittels, beispielsweise eines Luftfahrzeugs, vorgesehen sein. Die Sauerstoffmaske 2 weist einen Halterahmen 4 mit einem ersten Quersteg 6 und einem zweiten Quersteg 8 auf. Zusätzlich ist in diesem Ausführungsbeispiel ein dritter Quersteg 10 vorgesehen, der sich zwischen dem ersten Quersteg 6 und dem zweiten Quersteg 8 befindet. Die Querstege 6, 8 und 10 sind in zwei einander gegenüberliegenden seitlichen Haltern 12 angeordnet und dazu ausgebildet, einen Stoff 14 aufzuspannen. Der Stoff 14 ist im Wesentlichen gasundurchlässig und bildet zusammen mit den Querstegen 6, 8 und 10 die eigentliche, an das Gesicht der Person angepasste Maske.

Zumindest der erste Quersteg 6 ist flexibel. In dem gezeigten Ausführungsbeispiel sind auch die beiden übrigen Querstege 8 und 10 flexibel und erlauben eine relativ freie Formgebung. Die Querstege 6, 8 und 10 sind in den Haltern 12 verschiebbar gelagert. Hierzu weisen die seitlichen Halter 12 für jeden Quersteg 6, 8 und 10 einen geradlinigen Hohlraum 16 auf, in den der betreffende Quersteg 6, 8 und 10 eingesteckt ist. Durch ein Auseinanderziehen der seitlichen Halter 12 nach außen rutschen die Querstege 6 teilweise aus den Hohlräumen 16 heraus, so dass die Breite der Sauerstoffmaske 2 dadurch bedarfsweise vergrößert wird.

In dem Stoff 14 befindet sich ein Ausatemventil 18, das dazu vorgesehen ist, verbrauchte Luft aus der Maske herauszuführen. Eine Sauerstoffzuführungsleitung 20 erstreckt sich von einer hier nicht dargestellten Sauerstoffquelle durch den Stoff 14 und erzeugt auf einer der Person zugewandten Innenseite 22 (in der Zeichnungsebene verdeckt) eine sauerstoffangereicherte Atmosphäre.

Des Weiteren weist die Sauerstoffmaske 2 ein Kopfband 24 auf, welches an den seitlichen Haltern 12 angebracht ist und die Sauerstoffmaske 2 an dem Kopf einer Person sichern kann. Wie an schematisch angedeuteten Anschlussstellen 26 in Form von länglichen Vertiefungen gezeigt ist, kann auch das Kopfband 24 aus den seitlichen Haltern 12 etwas herausgezogen werden, um dessen effektive Länge zu vergrößern.

Fig. 2 zeigt in drei Darstellungen I, II und III die Sauerstoffmaske 2 in unterschiedlichen Zuständen. In der Darstellung I entspricht die Sauerstoffmaske 2 im Wesentlichen der Darstellung aus Fig. 1. Die Querstege 6, 8 und 10 weisen relativ große Abstände zueinander auf, so dass von einem größeren Gesicht auszugehen ist. In II sind die Abstände zwischen den Querstegen 6, 8 und 10 gering. Die Flexibilität der Querstege 6, 8 und 10 erlaubt ein sehr leichtes Verformen, so dass dadurch unter anderem der Abstand zwischen den Querstegen variabel ist. Hier ist beispielhaft gezeigt, dass der erste Quersteg 6 einen Bereich 28 aufweist, der zum Anpassen an die Nase einer Person vorgeformt ist bzw. eine vorgeformte Krümmung aufweist. In III wird ein geringer Abstand der seitlichen Haltern zueinander gezeigt, gleichzeitig ein geringer Abstand der Querstege 6, 8 und 10 zueinander. Die Sauerstoffmaske 2 könnte sich in diesem Zustand insbesondere für die Versorgung von Babys oder Kleinkindern eignen.

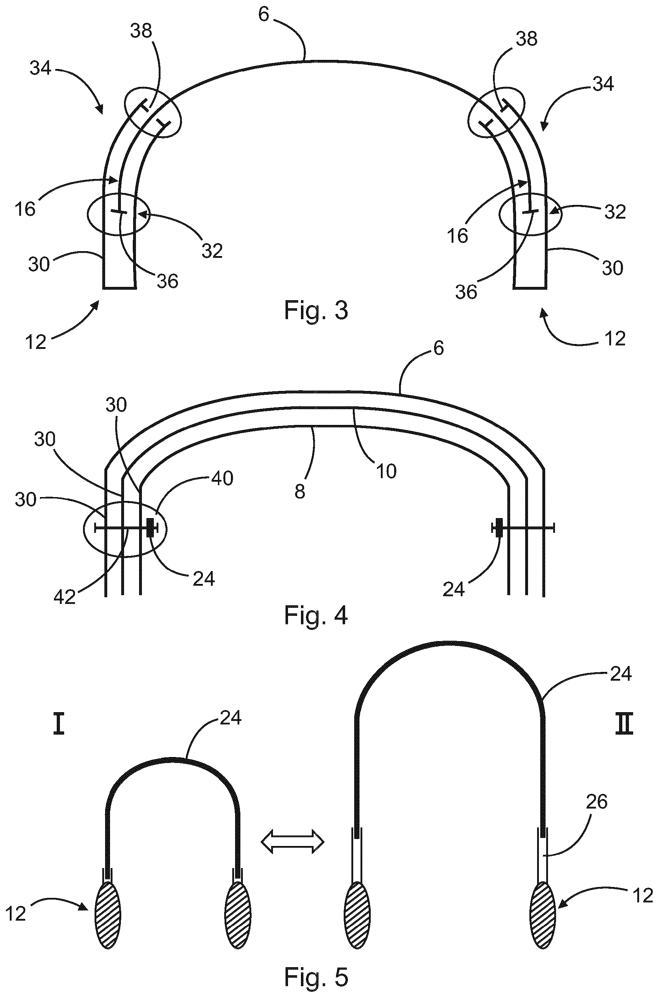

Fig. 3 zeigt ein Detail eines seitlichen Halters 12 und des ersten Querstegs 6. Die Ausführungen können auch auf die anderen Querstege 8 und 10 angewandt werden. Beispielhaft weist der seitliche Halter 12 mehrere Halteabschnitte 30 auf, in denen jeweils der vorangehend genannte Hohlraum 16 ausgebildet ist. Ein Ende 32 des ersten Querstegs 6 verläuft in dem Hohlraum 30 und kann eine variable Einstecktiefe einnehmen. Um zu verhindern, dass das Ende 32 vollständig aus dem Hohlraum 16 herausgezogen wird, ist eine Begrenzungseinrichtung 34 vorgesehen. Diese ist in Form einer endseitigen Aufdickung 36 an dem ersten Quersteg 6 sowie einer endseitigen Verjüngung 38 an dem Hohlraum 16 gebildet. Wird der erste Quersteg 6 aus dem Hohlraum 30 nach außen gezogen, geraten die endseitige Aufdickung 36 und die endseitige Verjüngung 38 des Hohlraum 16 in einen Flächenkontakt und verhindern eine weitergehende Bewegung. Dies ist auf beiden Seiten des ersten Querstegs 6 vorgesehen, d.h. an beiden seitlichen Haltern 12 bzw. daran angeordneten Halteabschnitten 30.

Fig. 4 zeigt die drei Querstege 6, 8 und 10, die jeweils in einem Halteabschnitt 30 angeordnet sind. Die drei Halteabschnitte 30 sind durch ein Gelenk 40 miteinander verbunden, das in dem gezeigten Fall ein stiftartiges Bauteil 42 aufweist und sich durch die drei Halteabschnitte 30 hindurch erstreckt. Die Halteabschnitte 30 können dann unterschiedlich weit aufgefächert werden und so die von den Querstegen 6, 8 und 10 überstrichene Fläche vergrößern oder verkleinern. An dem Gelenk 40 könnte auch das Kopfband 24 befestigt sein.

Fig. 5 zeigt das Kopfband 24 in zwei unterschiedlichen Darstellungen I und II. In I ist das Kopfband 24 durch Einschieben in die seitlichen Halter 12 bzw. die Anschlussstellen 26 verkürzt, während bei II das Kopfband 24 herausgezogen ist. Damit kann eine Anpassung an unterschiedliche Kopfgrößen vorgenommen werden.

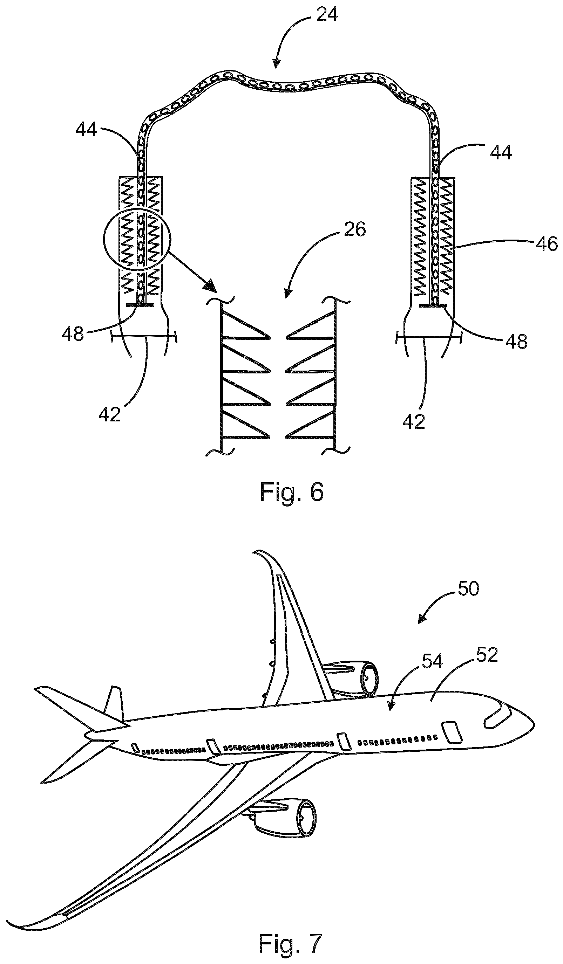

Fig. 6 zeigt beispielhaft, dass das Kopfband 24 einen versteiften Endabschnitt 44 aufweist, das in der Anschlussstelle 26 steckt. In dem dort vorliegenden Hohlraum sind beispielhaft sägezahnartige Vorsprünge 46 vorgesehen, die auf die versteiften Abschnitte 44 radial einwirken. Hierdurch wird ein gewisser mechanischer Widerstand erzeugt. Es ist weiterhin denkbar, dass hier ein inkrementelles Verschieben erreicht wird, in dem die versteiften Abschnitte 44 mit Rillen versehen sind, welche mit den Vorsprüngen 46 in Eingriff geraten können. Um zu verhindern, dass die versteiften Abschnitte 44 vollständig herausgezogen werden, sind Endanschläge 48 vorgesehen. Diese können mit dem innersten Vorsprüngen 46 in Flächenkontakt geraten, um so ein Herausziehen zu begrenzen.

Fig. 7 zeigt schließlich ein Verkehrsflugzeug 50, das einen Rumpf 52 mit einer darin ausgebildeten Kabine 54 aufweist. Dort können mehrere Sauerstoffmasken 2 angeordnet sein.

Ergänzend sei darauf hingewiesen, dass "aufweisend" keine anderen Elemente oder Schritte ausschließt, und "ein" oder "eine" keine Vielzahl ausschließt. Ferner sei darauf hingewiesen, dass Merkmale, die mit Verweis auf eines der obigen Ausführungsbeispiele beschrieben worden sind, auch in Kombination mit anderen Merkmalen anderer oben beschriebener Ausführungsbeispiele verwendet werden können. Bezugszeichen in den Ansprüchen sind nicht als Einschränkung anzusehen.

### BEZUGSZEICHEN

- 2: Sauerstoffmaske
- 4: Halterahmen
- 6: erster Quersteg
- 8: zweiter Quersteg
- 10: dritter Quersteg
- 12: seitlicher Halter
- 14: Stoff
- 16: Hohlraum
- 18: Ausatemventil
- 20: Sauerstoffzuführungsleitung
- 22: Innenseite
- 24: Kopfband
- 26: Anschlussstelle / Vertiefung
- 28: Bereich mit vorgeformter Krümmung
- 30: Halteabschnitt
- 32: Ende
- 34: Begrenzungseinrichtung
- 36: Aufdickung
- 38: Verjüngung
- 40: Gelenk
- 42: stiftartiges Bauteil
- 44: versteifter Endabschnitt
- 46: Vorsprung
- 48: Endanschlag
- 50: Verkehrsflugzeug / Luftfahrzeug
- 52: Rumpf
- 54: Kabine

## Patentansprüche

1. Sauerstoffmaske (2) zum Zuführen von Sauerstoff an eine Person, aufweisend:
- einen Halterahmen (4), der mindestens einen Quersteg (6, 8, 10) aufweist,
- einen auf dem Halterahmen (4) angeordneten und von dem mindestens einen Quersteg (6, 8, 10) aufspannbaren, im Wesentlichen gasundurchlässigen Stoff (14),
- ein an dem Stoff (14) angeordnetes und den Stoff (14) durchdringendes Ausatemventil (18), und
- eine Sauerstoffzuführungsleitung (20) zum Bereitstellen von Sauerstoff an einer Innenseite (22) der Sauerstoffmaske (2),
wobei der mindestens eine Quersteg (6, 8, 10) flexibel ist, und
wobei der Abstand der seitlichen Halter (12) zueinander zumindest zwischen einem ersten und einem zweiten Abstand einstellbar ist,
**dadurch gekennzeichnet, dass** die Sauerstoffmaske (2) ein mit dem Halterahmen (4) verbundenes Kopfband (24) aufweist, und
dass der mindestens eine Quersteg (6, 8, 10) in zwei einander gegenüberliegenden seitlichen Haltern (12) angeordnet ist.

2. Sauerstoffmaske (2) nach Anspruch 1,
wobei der mindestens eine Querstege (6, 8, 10) an oder in den seitlichen Haltern (12) verschiebbar gelagert ist.

3. Sauerstoffmaske (2) nach Anspruch 1 oder 2,
wobei mindestens einer der seitlichen Halter (12) für den mindestens einen Quersteg (6, 8, 10) einen geradlinigen Hohlraum (16) aufweist, der auf den betreffenden Quersteg (6, 8, 10) angepasst ist, so dass der betreffende Quersteg (6, 8, 10) unterschiedlich tief in den Hohlraum (16) einschiebbar ist.

4. Sauerstoffmaske (2) nach Anspruch 3,
wobei der Hohlraum (16) und der betreffende Quersteg (6, 8, 10) eine Begrenzungseinrichtung (34) aufweisen, die dazu ausgebildet ist, ein vollständiges Herausziehen des Querstegs (6, 8, 10) zu verhindern.

5. Sauerstoffmaske (2) nach Anspruch 4,
wobei die Begrenzungseinrichtung (34) in Form einer endseitigen Aufdickung (36) des betreffenden Querstegs (6, 8, 10) und einer endseitigen Verjüngung (38) des Hohlraums (16) gebildet ist.

6. Sauerstoffmaske (2) nach einem der vorhergehenden Ansprüche,
wobei der Stoff zwischen einem ersten Quersteg (6) und einem zweiten Quersteg (8) aufspannbar ist.

7. Sauerstoffmaske (2) nach Anspruch 6,
aufweisend einen dritten Quersteg (10), der zwischen dem ersten Quersteg (6) und dem zweiten Quersteg (8) angeordnet ist.

8. Sauerstoffmaske (2) nach einem der vorhergehenden Ansprüche,
wobei die seitlichen Halter (12) aus einem elastischen Material gebildet sind.

9. Sauerstoffmaske (2) nach einem der vorhergehenden Ansprüche,
wobei einer des mindestens einen Querstegs (6, 8, 10) bereichsweise eine vorgeformte Krümmung (28) aufweist, die zum Anschmiegen an die Nase der Person geformt ist.

10. Sauerstoffmaske (2) nach Anspruch 6 oder 7,
wobei die seitlichen Halter (12) mehrere Halteabschnitte (30) zum Aufnehmen jeweils eines Querstegs (6, 8, 10) aufweisen, und
wobei die Halteabschnitte (30) an einem Gelenk (40) angeordnet sind, das dazu ausgebildet ist, die Halteabschnitte (30) zum Anpassen an die Person variabel zueinander aufgefächert zu werden.

11. Sauerstoffmaske (2) nach einem der vorhergehenden Ansprüche,
wobei die seitlichen Halter (12) und der mindestens eine Quersteg (6, 8, 10) aus Silikon, einem silikonartigen Material, einem Elastomer oder einem gummiartigen Material ausgebildet sind.

12. Sauerstoffmaske (2) nach einem der vorhergehenden Ansprüche,
wobei das Kopfband (24) zwei steife Endabschnitte (44) aufweist, die jeweils in einer verzahnten Vertiefung (26) stecken, wobei das Kopfband (24) und die verzahnten Vertiefungen (26) dazu ausgebildet sind, eine effektive Länge des Kopfbands (24) inkrementell zu ändern.

13. Sauerstoffmaske (2) nach einem der vorhergehenden Ansprüche,
wobei die Sauerstoffzuführungsleitung (20) durch den Stoff (14) zu der Innenseite (22) der Sauerstoffmaske (2) ragt.

14. System zum Bereitstellen von Sauerstoff an Sauerstoffmasken (2) in einem Luftfahrzeug (50), aufweisend:
- eine Sauerstoffquelle und
- eine oder mehrere Sauerstoffmasken (2) nach einem der vorhergehenden Ansprüche,
wobei die Sauerstoffzuführungsleitung (20) der Sauerstoffmasken (2) jeweils mit der Sauerstoffquelle verbindbar sind.

15. Luftfahrzeug (50), aufweisend eine Passagierkabine (54) und mindestens ein System nach Anspruch 14.

## Claims

1. Oxygen mask (2) for supplying oxygen to a person, comprising:
- a holding frame (4) which has at least one cross-member (6, 8, 10),
- a substantially gas-impermeable material (14) which is arranged on the holding frame (4) and is clampable by the at least one cross-member (6, 8, 10),
- an exhalation valve (18) which is arranged on the material (14) and penetrates the material (14), and
- an oxygen supply line (20) for providing oxygen on an inner side (22) of the oxygen mask (2),
wherein the at least one cross-member (6, 8, 10) is flexible, and
wherein the distance of the lateral holders (12) from one another is adjustable at least between a first and a second distance,
**characterized in that** the oxygen mask (2) has a head band (24) which is connected to the holding frame (4), and
**in that** the at least one cross-member (6, 8, 10) is arranged in two mutually opposite lateral holders (12).

2. Oxygen mask (2) according to claim 1,
wherein the at least one cross-member (6, 8, 10) is mounted movably on or in the lateral holders (12).

3. Oxygen mask (2) according to claim 1 or 2,
wherein at least one of the lateral holders (12) for the at least one cross-member (6, 8, 10) has a rectilinear cavity (16) which is adapted to the respective cross-member (6, 8, 10), such that the respective cross-member (6, 8, 10) can be inserted into the cavity (16) to varying depths.

4. Oxygen mask (2) according to claim 3,
wherein the cavity (16) and the respective cross-member (6, 8, 10) have a limiting device (34) which is configured to prevent the cross-member (6, 8, 10) from being pulled out completely.

5. Oxygen mask (2) according to claim 4,
wherein the limiting device (34) is made in the form of an end-side thickened portion (36) of the respective cross-member (6, 8, 10) and an end-side tapered portion (38) of the cavity (16).

6. Oxygen mask (2) according to one of the preceding claims,
wherein the material is clampable between a first cross-member (6) and a second cross-member (8).

7. Oxygen mask (2) according to claim 6,
comprising a third cross-member (10) which is arranged between the first cross-member (6) and the second cross-member (8).

8. Oxygen mask (2) according to one of the preceding claims,
wherein the lateral holders (12) are formed from an elastic material.

9. Oxygen mask (2) according to one of the preceding claims,
wherein one of the at least one cross-member (6, 8, 10) has, in regions, a pre-formed curvature (28) which is shaped to adapt to the nose of the person.

10. Oxygen mask (2) according to claim 6 or 7,
wherein the lateral holders (12) have a plurality of holding portions (30) for receiving in each case one cross-member (6, 8, 10), and
wherein the holding portions (30) are arranged on a joint (40) which is configured to allow the holding portions (30) to be fanned out variably relative to one another in order to adapt to the person.

11. Oxygen mask (2) according to one of the preceding claims,
wherein the lateral holders (12) and the at least one cross-member (6, 8, 10) are formed from silicone, a silicone-like material, an elastomer or a rubber-like material.

12. Oxygen mask (2) according to one of the preceding claims,
wherein the head band (24) has two stiff end portions (44) which are each inserted into a toothed depression (26), wherein the head band (24) and the toothed depressions (26) are configured to change an effective length of the head band (24) incrementally.

13. Oxygen mask (2) according to one of the preceding claims,
wherein the oxygen supply line (20) projects through the material (14) to the inner side (22) of the oxygen mask (2).

14. System for providing oxygen to oxygen masks (2) in an aircraft (50), comprising:
- an oxygen source, and
- one or more oxygen masks (2) according to one of the preceding claims,
wherein the oxygen supply line (20) of the oxygen masks (2) can be connected in each case to the oxygen source.

15. Aircraft (50), comprising a passenger cabin (54) and at least one system according to claim 14.

## Revendications

1. Masque à oxygène (2) pour alimenter de l'oxygène à une personne, comprenant:
- un cadre de support (4) qui comprend au moins une traverse (6, 8, 10),
- un tissu (14) sensiblement imperméable aux gaz, disposé sur le cadre de support (4) et pouvant être tendue par l'au moins une traverse (6, 8, 10),
- une valve d'expiration (18) disposée sur le tissu (14) et pénétrant le tissu (14), et
- une conduite d'alimentation en oxygène (20) pour fournir de l'oxygène sur un côté intérieur (22) du masque à oxygène (2),
dans lequel l'au moins une traverse (6, 8, 10) est flexible, et
dans lequel la distance des supports latéraux (12) les uns par rapport aux autres est réglable au moins entre une première et une deuxième distance,
**caractérisé en ce que** le masque à oxygène (2) comprend une bande de tête (24) reliée au cadre de support (4), et
**en ce que** l'au moins une traverse (6, 8, 10) est disposée dans deux supports latéraux (12) opposés l'un à l'autre.

2. Masque à oxygène (2) selon la revendication 1,
dans lequel l'au moins une traverse (6, 8, 10) est montée de manière déplaçable sur ou dans les supports latéraux (12).

3. Masque à oxygène (2) selon la revendication 1 ou 2,
dans lequel au moins l'un des supports latéraux (12) pour l'au moins une traverse (6, 8, 10) comprend un espace creux rectiligne (16) qui est adapté à la traverse (6, 8, 10) concernée de sorte que la traverse (6, 8, 10) concernée peut être insérée à des profondeurs différentes dans l'espace creux (16).

4. Masque à oxygène (2) selon la revendication 3,
dans lequel l'espace creux (16) et la traverse (6, 8, 10) concernée comprennent un dispositif de limitation (34) qui est configuré pour empêcher un retrait complet de la traverse (6, 8, 10).

5. Masque à oxygène (2) selon la revendication 4,
dans lequel le dispositif de limitation (34) est formé sous la forme d'un épaississement côté extrémité (36) de la traverse (6, 8, 10) concernée et d'un rétrécissement côté extrémité (38) de l'espace creux (16).

6. Masque à oxygène (2) selon l'une des revendications précédentes,
dans lequel le tissu peut être tendu entre une première traverse (6) et une deuxième traverse (8).

7. Masque à oxygène (2) selon la revendication 7,
comprenant une troisième traverse (10) qui est disposée entre la première traverse (6) et la deuxième traverse (8).

8. Masque à oxygène (2) selon l'une des revendications précédentes,
dans lequel les supports latéraux (12) sont formés d'un matériau élastique.

9. Masque à oxygène (2) selon l'une des revendications précédentes,
dans lequel l'une de l'au moins une traverse (6, 8, 10) présente par endroits une courbure préformée (28) qui est formée pour épouser le nez de la personne.

10. Masque à oxygène (2) selon la revendication 6 ou 7,
dans lequel les supports latéraux (12) comprennent plusieurs sections de retenue (30) pour recevoir respectivement une traverse (6, 8, 10), et
dans lequel les sections de retenue (30) sont disposées sur une articulation (40) qui est conçue pour être déployée de manière variable les unes par rapport aux autres pour adapter les sections de retenue (30) à la personne.

11. Masque à oxygène (2) selon l'une des revendications précédentes,
dans lequel les supports latéraux (12) et l'au moins une traverse (6, 8, 10) sont formés de silicone, d'un matériau de type silicone, d'un élastomère ou d'un matériau de type caoutchouc.

12. Masque à oxygène (2) selon l'une des revendications précédentes,
dans lequel la bande de tête (24) comprend deux sections d'extrémité (44) rigides qui s'insèrent respectivement dans un creux denté (26), la bande de tête (24) et les creux dentés (26) étant configurés pour modifier de manière incrémentielle une longueur effective de la bande de tête (24).

13. Masque à oxygène (2) selon l'une des revendications précédentes,
dans lequel la conduite d'alimentation en oxygène (20) fait saillie à travers le tissu (14) vers le côté intérieur (22) du masque à oxygène (2).

14. Système pour fournir de l'oxygène à des masques à oxygène (2) dans un aéronef (50), comprenant :
- une source d'oxygène, et
- un ou plusieurs masques à oxygène (2) selon l'une des revendications précédentes,
dans lequel la conduite d'alimentation en oxygène (20) des masques à oxygène (2) peut être reliée respectivement à la source d'oxygène.

15. Aéronef (50) comprenant une cabine passagers (54) et au moins un système selon la revendication 14.
